# EUROPÄISCHE PATENTSCHRIFT

(11) **EP 1 567 516 B1**
(45) Veröffentlichungstag und Bekanntmachung des Hinweises auf die Patenterteilung: **07.06.2006**
(21) Anmeldenummer: 03779948.3
(22) Anmeldetag: 17.11.2003
(51) Int. Cl.: C07D 339/04, A61K 31/385, A61P 5/50

(54) **VERFAHREN ZUR REINIGUNG VON LIPONSÄURE**
METHOD FOR THE PURIFICATION OF LIPONIC ACID
PROCEDE DE PURIFICATION D'ACIDE LIPOIQUE

(30) Priorität: 26.11.2002 DE 10255242
(43) Veröffentlichungstag der Anmeldung: 31.08.2005
(73) Patentinhaber: BASF Aktiengesellschaft, 67056 Ludwigshafen (DE)
(72) Erfinder: MAIER, Ulrike, 68161 Mannheim (DE); KLATT, Martin Jochen, 67098 Bad Dürkheim (DE)
(86) Internationale Anmeldenummer: PCT/EP2003/012819
(87) Internationale Veröffentlichungsnummer: WO 2004/048362

(56) Entgegenhaltungen:
- DE-A- 10 044 000
- CHEMICAL ABSTRACTS, vol. 71, no. 25, 1969 Columbus, Ohio, US; abstract no. 119752c, NAKATA,TARO: "BIOLOGICALLY ACTIVE LIPOIC ACID-LIKE SUBSTANCES IN URINE.II.PURIFICATION" Seite 24; Spalte 2; XP002268589 & BITAMIN, Bd. 40, Nr. 3, 1969, Seiten 185-192, ENG

## Beschreibung

Die vorliegende Erfindung betrifft ein Verfahren zur Reinigung von racemischer oder nicht-racemischer Liponsäure. Insbesondere betrifft die Erfindung ein Verfahren zur Reinigung von R- oder S-Liponsäure, die einen Oligomerenanteil im Bereich von etwa 0,1 bis etwa 1,0 Gew.% aufweist.

Ferner betrifft die Erfindung die Verwendung der in der erfindungsgemäßen Weise gereinigten Liponsäure in Arzneimitteln, Kosmetika und Lebensmitteln.

Dihydroliponsäure und Liponsäure sind natürlich vorkommende Substanzen, denen eine besondere Bedeutung im Zellstoffwechsel zukommt. R-Liponsäure spielt als Coenzym, z. B. der Pyruvatdehydrogenase, eine zentrale Rolle bei der Energiegewinnung. R-Liponsäure wird zur vollen Entfaltung ihrer sehr guten antioxidativen Eigenschaften im Stoffwechsel zu Dihydroliponsäure aktiviert. Dihydroliponsäure und Liponsäure können, da sie in-vivo ineinander überführt werden, für die gleichen Einsatzgebiete verwendet werden. R-Liponsäure beeinflusst altersbedingte Veränderungen im Stoffwechsel vorteilhaft und ist daher auch im kosmetischen Bereich von Interesse.

Liponsäure und Dihydroliponsäure können als Nutraceutical im Lebensmittelbereich eingesetzt werden. Auch der Einsatz von Dihydroliponsäure und/oder Liponsäure als Pharmakon ist möglich. Beispielsweise ist bekannt, dass R-Liponsäure die Insulinsensitivität erhöht und damit als Antidiabetikum, auch für die Verhinderung und Linderung von diabetischen Spätschäden, verwendet werden kann. Weiterhin können Liponsäure oder Dihydroliponsäure oder deren Derivate zur Behandlung von Glukosestoffwechselstörungen (z.B. im ZNS), bei Insulinresistenz, Krebs und bei Hörstörungen eingesetzt werden.

Aus der Literatur (G. Bringmann, D. Herzberg, G. Adam, F. Balkenhohl, J. Paust, Z. Naturforschung 1999, 54b, 665-661; B. Adger et al., Bioorg, Med. Chem. 1997, 5, 253-61; J.S. Yadav, S. Mysorekar, K. Garyali, J. Scientific & Industrial Res. 1990, 49, 400-409; A.S. Gopalan, H.K. Jacobs, Tetrahedron Lett 1989, 42, 5705; M.H. Brookes, B.T. Golding, A.T. Hudson, Perkin Transaction 1, 1988, 9-12; M.H. Brookes, B.T. Golding, D.A. Howes, A.T. Hudson, Chemical Communication 1983, 1051-53; JP 1960-35704; EP-A-543088; EP-A-487 986; DE-A-100 44 000 und DE-A-100 59 718) sind zahlreiche Methoden der Herstellung von optisch reiner R- und S-Liponsäure bzw. Dihydroliponsäure bekannt.

Da Liponsäure und Dihydroliponsäure auch am Menschen eingesetzt werden sollen, sind möglichst reine Produkte erwünscht, deren einfache und ökonomische Herstellung in großen Mengen sicherzustellen ist.

Als besonders problematisch ist in diesem Zusammenhang die Verunreinigung der gewonnenen Liponsäure durch charakteristische Verunreinigungen zu betrachten, die durch Oligomerisierung bzw. Polymerisierung der Liponsäure selbst entstehen. Bereits geringe Mengen dieser Oligomeren im Produkt führen zu schwerwiegenden Komplikationen bei der Weiterverarbeitung und können im Hinblick auf eine pharmazeutische Nutzung der so hergestellten Liponsäure nicht toleriert werden.

Die DE-A-100 44 000 und DE-A-100 59 718 beschreiben ein Verfahren zur Reinigung von durch Oxidation von Dihydroliponsäure gewonnener Liponsäure durch Klärfiltration, welches für Versuche im Labormaßstab geeignet ist.

Die großtechnische Herstellung von Liponsäure mit Oligomerenanteilen von weniger als 1,0 Gew.% hingegen stellt ein bislang nicht zufriedenstellend gelöstes Problem dar.

Aufgabe der vorliegenden Erfindung war die Entwicklung eines Verfahrens zur Reinigung von Liponsäure, welches auch in großtechnischem Maßstab ökonomisch anwendbar ist.

Es wurde nun ein Verfahren zur Reinigung von Liponsäure gefunden, das dadurch gekennzeichnet ist, dass man eine Lösung von Liponsäure mit der bezogen auf die Masse an zu reinigender Liponsäure mindestens 0,1-fachen Menge eines Adsorptionsmittels versetzt und anschließend das Adsorptionsmittel abtrennt. Auf diese Weise kann racemische oder nicht-racemische Liponsäure mit einem Oligomerenanteil unter 1 Gew.%, beispielsweise 0,1 bis 1 Gew.% zur Verfügung gestellt werden.

Zur Herstellung von Lösungen für den erfindungsgemäßen Reinigungsprozess von Liponsäure eignen sich wenig polare organische Lösungsmittel, insbesondere aliphatische, gegebenenfalls heteroatomsubstituierte Kohlenwasserstoffe mit einem bis zwölf Kohlenstoffatomen wie z.B. Pentan, Hexan, Cyclohexan, Heptan oder Dichlorethan oder aromatische, gegebenenfalls alkyl- bzw. heteroatomsubstituierte Kohlenwaserstoffe wie Toluol, Ethylbenzol oder Chlorbenzol oder Gemische aus den genannten Lösemittelklassen.

Bevorzugte Lösungsmittel für den erfindungsgemäßen Reinigungsprozess sind aliphatische Kohlenwasserstoffe mit einem bis zwölf Kohlenstoffatomen und alkylsubstituierte aromatische Kohlenwasserstoffe mit bis zu zwölf Kohlenstoffatomen.

Besonders bevorzugte Lösungsmittel sind Pentan, Hexan, Cyclohexan, Heptan, Oktan, Toluol und Ethylbenzol, ganz besonders bevorzugt Heptan und Toluol oder Gemische von mindestens zwei der genannten Lösungsmittel.

Als Adsorptionsmittel eignen sich handelsübliche Adsorptionsmittel wie Kieselgele, neutrale Aluminiumoxide, basische Aluminiumoxide sowie Gemische der genannten Adsorptionsmittel, bevorzugt Kieselgele.

Besonders bevorzugte Adsorptionsmittel sind Kieselgel 60 mit einer Korngröße von 0,04-0,063 mm, Kieselgel 40 (Korngröße 0,015 - 0,035 mm) und Kieselgel 100 (Korngröße 0,063 - 0,2 mm).

Die Menge des im erfindungsgemäßen Reinigungsverfahren einzusetzenden Adsorptionsmittels richtet sich nach der Menge der zu reinigenden Liponsäure und beträgt im allgemeinen mindestens 10 Gew.% der Menge an zu reinigender Liponsäure. Dies gilt insbesondere bei Verwendung des besonders bevorzugten Adsorptionsmittels Kieselgel. Besonders bevorzugt ist dabei die Verwendung von mindestens 15 Gew.% Kieselgel bezogen auf die Menge an zu reinigender Liponsäure. Ganz besonders bevorzugt verwendet man 20 bis 50 Gew.% Kieselgel bezogen auf die Menge an zu reinigender Liponsäure.

Zur Abtrennung des Adsorptionsmittels eignen sich alle dem Fachmann geeignet erscheinende Trennverfahren. Zur Abtrennung des als Adsorptionsmittel besonders bevorzugt eingesetzten Kieselgels eignet sich insbesondere die Durchführung einer Filtration.

Das erfindungsgemäße Verfahren eignet sich zur Reinigung von racemischer wie nicht-racemischer Liponsäure. Unter nicht-racemischer Liponsäure sind in diesem Zusammenhang alle Gemische von R- und S-Liponsäure zu verstehen, in denen die beiden Enantiomere nicht zu gleichen Teilen vorliegen.

Bevorzugt werden durch das erfindungsgemäße Verfahren jedoch R- bzw.- S-Liponsäure mit einem Enantiomerenüberschuss (ee) von jeweils größer 80% ee, besonders bevorzugt größer 90% ee, ganz besonders bevorzugt größer 95% ee gereinigt. Am meisten bevorzugt werden R-bzw. S-Liponsäure mit einem Enantiomerenüberschuss von jeweils größer 99% ee gereinigt. Das erfindungsgemäße Reinigungsverfahren eignet sich zur Herstellung von chemisch reiner R-bzw. S-Liponsäure. Besonders eignet es sich zur Herstellung von R- bzw. S-Liponsäure mit einer chemischen Reinheit von mindestens 99%, ganz besonders mindestens 99,5%. Es eignet sich insbesondere zur Herstellung von R- bzw. S-Liponsäure mit einem Gehalt der als Verunreinigung problematischen Oligomeren der Liponsäure von maximal 0,5 Gew.%. Ganz besonders eignet sich das erfindungsgemäße Reinigungsverfahren zur Herstellung von R- bzw. S-Liponsäure mit einem Oligomerenanteil von maximal 0,2 Gew.%.

Die zu reinigende Liponsäure kann vorgereinigt oder als festes oder teilweise gelöstes Rohprodukt eines vorangegangenen Verfahrensschrittes im erfindungsgemäßen Reinigungsverfahren eingesetzt werden. Das Reinigungsverfahren kann in jedem Maßstab durchgeführt werden. Insbesondere eignet es sich jedoch für die Reinigung von Liponsäure in großtechnischem Maßstab.

Über das Verfahren zur Reinigung von Liponsäure hinaus betrifft die Erfindung auch die Weiterverarbeitung der erfindungsgemäß gereinigten R- oder S-Liponsäure, z. B. in pharmakologisch verträgliche Salze, wie Alkali- und Erdalkalisalze oder z. B. das Trometamol-Salz (Internat. Freiname für Tris(hydroxymethyl)-aminomethan) der R-Liponsäure.

Darüber hinaus betrifft die Erfindung auch die Verwendung von durch das erfindungsgemäße Verfahren gereinigter, racemischer wie nicht-racemischer bzw. enantiomerenreiner Liponsäure in Arzneimitteln, in Kosmetika oder in Lebensmitteln, etwa als Nutraceutical. Dies umfasst die Formulierung von in erfindungsgemäßer Weise gereinigter racemischer oder nicht-racemischer Liponsäure, insbesondere von erfindungsgemäß gereinigter R- oder S-Liponsäure in einer pharmakologisch oder dermatologisch verträglichen Form.

Im Hinblick auf die vielfältigen Formulierungs- und Verwendungsmöglichkeiten der Liponsäure sei exemplarisch auf den Inhalt der DE-A-100 22 856 und der DE-A-100 27 875 (PCT/EP/01/06385) verwiesen.

Die folgenden Beispiele dienen der Veranschaulichung der Erfindung, ohne sie jedoch einzuschränken:

### Ausführungsbeispiel:

25,3 kg (250 mol) Triethylamin und 20 kg (100 mol) (6S)-6,8-Dihydroxyoctansäuremethylester wurden in 300 Liter Toluol vorgelegt und unter Kühlung 28,6 kg (250 mol) Methansulfonylchlorid zudosiert. Nach Abtrennung des Triethylammoniumhydrochlorids wurde die Hauptmenge des Lösemittels abdestilliert.

Anschließend wurde eine methanolische Lösung von 50,7 kg Natriumsulfid und 14,4 kg Schwefel zugegeben. Nach vollständigem Umsatz wurde mit Wasser verdünnt und 40 kg einer 12%-igen Lösung von NaBH₄ in 14 M NaOH (Borol-Lösung) zugegeben. Ein Teil des Lösemittelgemisches wurde abdestilliert und das verbliebene Reaktionsgemisch mit Schwefelsäure auf pH 4 gebracht und anschließend mit Toluol extrahiert. Die organische Phase wurde auf pH 9 gebracht und mit Toluol extrahiert.

Die so isolierte R-Dihydroliponsäure wurde mit Wasser aufgenommen, mit einer katalytischen Menge Eisen(II)-sulfat versetzt und bis zum vollständigen Umsatz mit Luft begast. Anschließend wurde mit Schwefelsäure angesäuert (pH 2) und mit Toluol extrahiert. Die toluolische Lösung wurde mit Heptan versetzt und mit leichtem Überdruck über einen mit 5 kg Kieselgel 60 gefüllten Druckfilter gedrückt.

Die filtrierte Lösung wurde abgekühlt und die dabei auskristallisierte R-Liponsäure durch Druckfiltration isoliert und im Stickstoffstrom getrocknet.

Man erhielt R-Liponsäure (ee > 99,9%) in einer Ausbeute von 73% d.Th. bzgl. (6S)-6,8-Di-hydroxyoctansäuremethylester. Der Gehalt an Oligomeren der Liponsäure wurde durch Gel-Permeations-Chromatographie (Säule: PL-Gel, Eluent THF) zu 0,3% bestimmt.

### Vergleichsbeispiel:

Das oben genannte Beispiel wurde bis zum Reinigungsschritt unter gleichen Bedingungen wiederholt. Die nach dem Ansäuern mit Schwefelsäure erhaltene toluolische Lösung wurde mit Heptan versetzt und mit leichtem Überdruck über einen mit 1 kg Kieselgel 60 gefüllten Druckfilter gedrückt.

Die filtrierte Lösung wurde abgekühlt und die dabei auskristallisierte R-Liponsäure durch Druckfiltration isoliert und im Stickstoffstrom getrocknet.

Man erhielt R-Liponsäure (ee > 99,9%) in einer Ausbeute von 73% d.Th. bzgl. (6S)-6,8-Dihydroxyoctansäuremethylester. Der Gehalt an Oligomeren der Liponsäure wurde durch Gel-Permeations-Chromatographie (Säule: PL-Gel, Eluent THF) zu 2,5% bestimmt.

## Patentansprüche

1. Verfahren zur Reinigung von Liponsäure, **dadurch gekennzeichnet, dass** man eine Lösung von Liponsäure mit der bezogen auf die Masse an zu reinigender Liponsäure mindestens 0,1-fachen Menge eines Adsorptionsmittels versetzt und anschließend das Adsorptionsmittel abtrennt.

2. Verfahren gemäß Anspruch 1, **dadurch gekennzeichnet, dass** die zu reinigende Liponsäure durch Oxidation von Dihydroliponsäure erhalten wurde.

3. Verfahren gemäß Anspruch 1 und 2, **dadurch gekennzeichnet, dass** man die bezogen auf die Masse an zu reinigender Liponsäure mindestens 0,2 bis 0,5-fache Menge eines Adsorptionsmittels verwendet.

4. Verfahren gemäß Anspruch 1 bis 3, **dadurch gekennzeichnet, dass** man als Adsorptionsmittel Kieselgel verwendet.

5. Verfahren gemäß Anspruch 1 bis 4, **dadurch gekennzeichnet, dass** man die zu reinigende Liponsäure in einem organischen Lösungsmittel oder in einem Gemisch mindestens zweier organischer Lösungsmittel löst.

6. Verfahren gemäß Anspruch 1 bis 5, **dadurch gekennzeichnet, dass** man die Liponsäure in Heptan oder Toluol oder einem Gemisch dieser Lösungsmittel löst.

7. Verfahren gemäß einem der Ansprüche 1 bis 6, **dadurch gekennzeichnet, dass** als Liponsäure racemische oder nicht-racemische Liponsäure eingesetzt wird.

8. Verwendung von gemäß einem der Ansprüche 1 bis 7 gereinigter Liponsäure in Arzneimitteln, Kosmetika und Lebensmitteln.

## Claims

1. A process for purifying lipoic acid, which comprises adding to a solution of lipoic acid at least 0.1 times the amount of an adsorbent, based on the mass of lipoic acid to be purified, and subsequently removing the adsorbent.

2. The process according to claim 1, wherein the lipoic acid to be purified has been obtained by oxidation of dihydrolipoic acid.

3. The process according to claim 1 or 2, wherein at least 0.2 to 0.5 times the amount of an adsorbent, based on the mass of lipoic acid to be purified, is used.

4. The process according to claim 1 to 3, wherein silica gel is used as adsorbent.

5. The process according to claim 1 to 4, wherein the lipoic acid to be purified is dissolved in an organic solvent or in a mixture of at least two organic solvents.

6. The process according to claim 1 to 5, wherein the lipoic acid is dissolved in heptane or toluene or a mixture of these solvents.

7. The process according to any of claims 1 to 6, wherein racemic or nonracemic lipoic acid is employed as lipoic acid.

8. The use of lipoic acid purified according to any of claims 1 to 7 in drugs, cosmetics and food products.

## Revendications

1. Procédé de purification d'acide lipoïque, **caractérisé en ce qu'**à une solution d'acide lipoïque on ajoute au moins 0,1 fois la quantité d'un agent adsorbant par rapport à la masse d'acide lipoïque à purifier et on isole ensuite l'agent adsorbant.

2. Procédé suivant la revendication 1, **caractérisé en ce que** l'acide lipoique à purifier a été obtenu par oxydation d'acide dihydrolipoïque.

3. Procédé suivant la revendication 1 ou 2, **caractérisé en ce qu'**on utilise au moins 0,2 à 0,5 fois la quantité d'un agent adsorbant par rapport à la masse d'acide lipoique à purifier.

4. Procédé suivant les revendications 1 à 3, **caractérisé en ce qu'**on utilise du gel de silice en tant qu'agent adsorbant.

5. Procédé suivant les revendications 1 à 4, **caractérisé en ce qu'**on dissout l'acide lipoïque à purifier dans un solvant organique ou dans un mélange d'au moins deux solvants organiques.

6. Procédé suivant les revendications 1 à 5, **caractérisé en ce qu'**on dissout l'acide lipoique dans de l'heptane ou du toluène ou dans un mélange de ces solvants.

7. Procédé suivant l'une des revendications 1 à 6, **caractérisé en ce que**, comme acide lipoïque, on met en oeuvre de l'acide lipoïque racémique ou non racémique.

8. Utilisation d'acide lipoïque purifié suivant l'une des revendications 1 à 7, dans des médicaments, des produits cosmétiques et des aliments.
